# EUROPEAN PATENT SPECIFICATION

(11) **EP 0 574 135 B1**
(45) Date of publication and mention of the grant of the patent: **18.11.1998**
(21) Application number: 93303644.4
(22) Date of filing: 11.05.1993
(51) Int. Cl.: C07K 5/08

(54) **Process for producing peptide derivatives and salts thereof**
Verfahren zur Herstellung von Peptidderivaten und ihrer Salze
Procédé de préparation de dérivés peptidiques et leurs corps salins

(30) Priority: 13.05.1992 JP 192653/92; 13.05.1992 JP 192654/92; 25.05.1992 JP 157459/92; 30.10.1992 JP 315640/92; 09.11.1992 JP 323599/92
(43) Date of publication of application: 15.12.1993
(73) Proprietor: JAPAN ENERGY CORPORATION, Minato-ku, Tokyo (JP)
(72) Inventor: Mimoto, Tsutomu, c/o Nikko Kyodo Co. Ltd., Toda-shi, Saitama (JP); Kisanuki, Sumitsugu, c/o Nikko Kyodo Co. Ltd., Toda-shi, Saitama (JP); Takahasihi, Osamu, c/o Nikko Kyodo Co. Ltd., Toda-shi, Saitama (JP); Kiso, Yoshiaki, Ibaraki-shi, Osaka (JP)
(74) Representative: Geering, Keith Edwin

(56) References cited:
- EP-A- 0 498 680
- CHEMICAL ABSTRACTS, vol. 117, no. 1, July 6, 1992 Columbus, Ohio, USA MIMOTO, TSUTOMO et al. "Design and synthesis of HIV protease inhibitors containing a hydroxymethyl- -carbonyl isostere as a transition state mimic." page 856-7, column 2, abstract-no. 8449h

## Description

### BACKGROUND OF THE INVENTION

### Field of the Invention:

The present invention relates to a process for producing peptide derivatives and salts thereof which are useful as human immunodeficiency virus (HIV) protease inhibitors.

### Description of the Related Art:

Heretofore, various efforts have been made for the therapy of acquired immunodeficiency syndrome (AIDS) and prevention of HIV infection by inhibiting the HIV protease. The present inventors have proposed peptide derivatives and salts thereof which inhibit the HIV protease [European Patent No. 490667]. The methods for producing said peptide derivatives and salts thereof, however, have been expensive and/or required toxic reagents, for example, Bop reagent.

### SUMMARY OF THE INVENTION

It is an object of the present invention to provide a convenient process for producing peptide derivatives and salts thereof which inhibit HIV protease without using expensive and/or toxic reagents.

The object of the present invention has been attained by the following process for producing peptide derivatives represented by the following formula (I) and salts thereof: 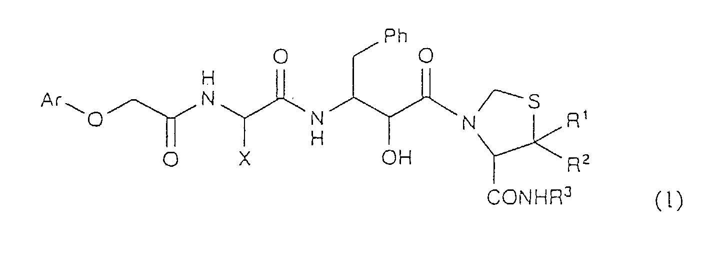 (wherein R¹ and R² represent a lower alkyl group or hydrogen atom, R³ represents a lower alkyl group, X represents methylthiomethyl, methanesulfonylmethyl, carbamoylmethyl, or a lower alkyl group, Ar represents an aryl group or a heteroaryl group). The process includes (a) condensation reaction (step A) of a peptide derivative represented by formula (2): 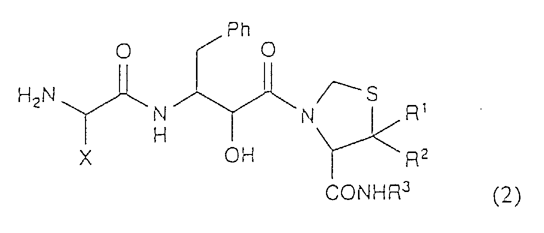 (wherein R¹, R², R³, and X are as defined above) with a carboxylic acid represented by the following formula (3): 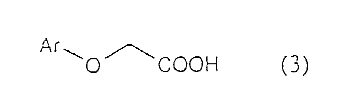 (wherein Ar is as defined above); or (b) a condensation reaction (step B') of a peptide derivative represented by the formula (4): 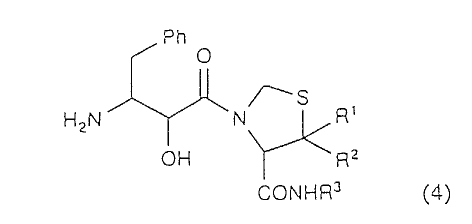 (wherein R¹, R², and R³ are as defined above) with a protected amino acid represented by the following formula (5'): 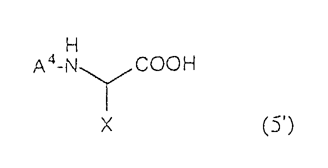 (wherein A⁴ represents an acyl group derived from the carboxylic acid of formula (3) and X is as defined above); each gives a peptide derivative represented by formula (1).

The object of the present invention also has been attained by the following process for producing peptide derivatives represented by formula (1) and salts thereof: 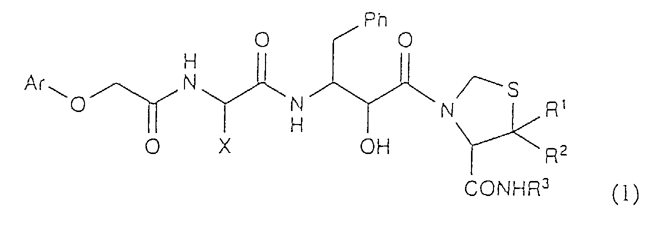 (wherein R¹, R², R³, X and Ar are as defined above) from compounds of formula (9) below. Formula (9) compound can be obtained by a process which comprises condensation reaction (step C) of a protected amino acid represented by the following formula (6): 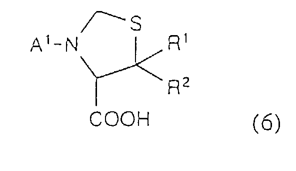 (wherein R¹ and R² represent a lower alkyl group or hydrogen atom, A¹ represents an amino protective group) with an amine represented by the following formula (7):

H₂N-R³ (7)

(wherein R³ represents a lower alkyl group) which gives a protected amino amide represented by the following formula (8): 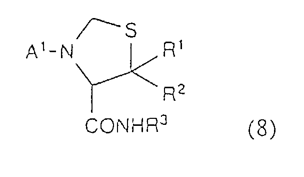 (wherein A¹, R¹, R², and R³ are as defined above), and deprotection (step D) of said protected amino amide which gives an amino amide represented by the following formula (9): 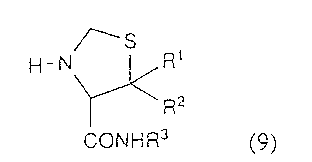 (wherein R¹, R², and R³ are as defined above). Compound (9) is converted to formula (1) derivative or salt thereof by condensation reaction (step E) of said amino amide (9) with a protected amino acid represented by the following formula (10): 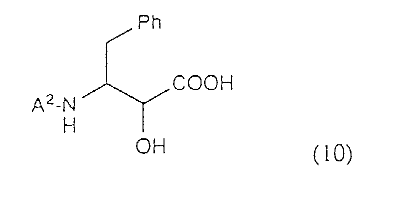 (wherein A² represents an amino protective group) which gives a protected peptide represented by the following formula (11): 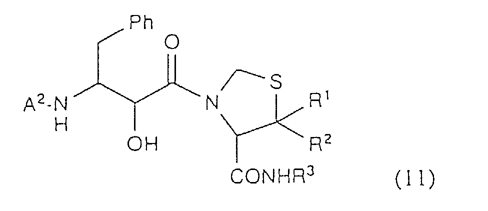 (wherein A², R¹, R², and R³ are as defined above), and deprotection (step F) of said protected peptide which gives a peptide represented by the following formula (4) 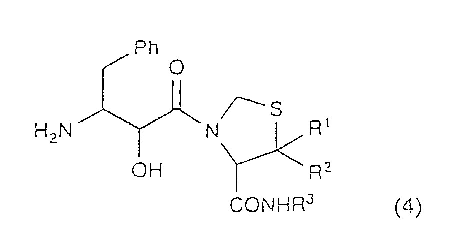 (wherein R¹, R², and R³ are as defined above), followed by (a) condensation reaction (step B) of said peptide with a protected amino acid represented by the following formula (5): 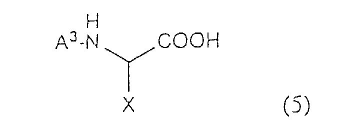 (wherein A³ represents an amino protective group, X represents methylthiomethyl, methanesulfonylmethyl, carbamoylmethyl, or a lower alkyl group) which gives a protected peptide represented by the following formula (12): 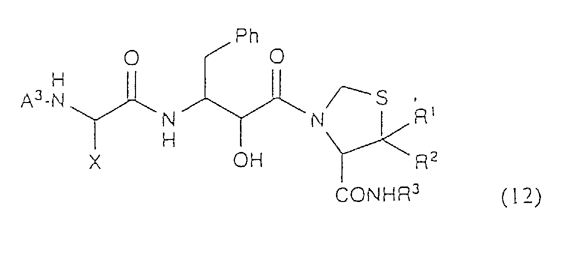 (wherein A³, R¹, R², R³, and X are as defined above), deprotection reaction (step G) of said protected peptide which gives a peptide represented by above formula (2), 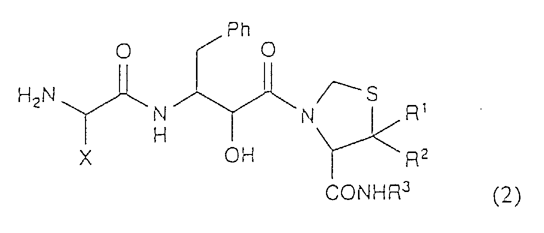 (wherein R¹, R², R³, and X are as defined above), and a condensation reaction (step A) of said peptide with a carboxylic acid represented by the following formula (3): 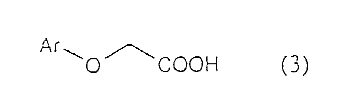 (wherein Ar represents an aryl group or a heteroalkyl group), or a condensation reaction (step B') of a peptide derivative represented by the following formula (4): 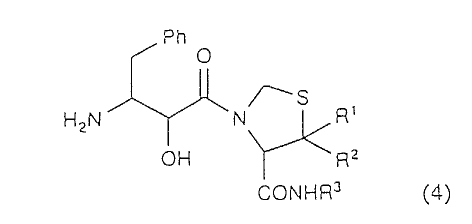 (wherein R¹, R², and R³ are as defined above) with a protected amino acid represented by the following formula (5'): 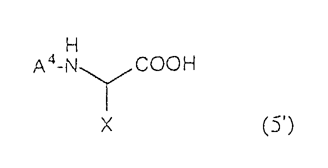 (wherein A⁴ and X are as defined above) which gives a peptide derivative represented by the formula (1).

Among the starting materials of the above process, a compound represented by formula (6) wherein A¹ represents *tert*-butoxycarbonyl may be produced by cyclization of a compound represented by the following formula (13): 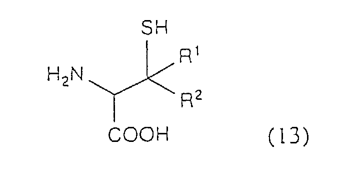 (wherein R¹ and R² are as defined above), or the salt thereof using formaldehyde and a subsequent one-pot amino-protection using di-*tert*-butyl dicarbonate (step I).

In addition, a compound represented by the formula (5) wherein X represents methylthiomethyl and A³ represents *tert*-butoxycarbonyl may be produced by methylation of cysteine and a subsequent one-pot amino-protection using di-*tert*-butyl dicarbonate (step II).

### DETAILED DESCRIPTION OF THE INVENTION

As a protective group of the present invention represented by A¹, A², and A³, amino protective groups which are generally used in peptide synthesis, for example p-methoxybenzyloxycarbonyl or *tert-*butoxycarbonyl, may be used. As a lower alkyl group represented by R¹, R², R³, and X, there may be mentioned methyl, ethyl, propyl, isopropyl, *n*-butyl, *sec*-butyl*, tert*-butyl, and amyl. As an aryl group represented by Ar, there may be mentioned phenyl, 1-naphthyl, 5-isoquinolyl, and 3-pyridyl.

The amino deprotection of the present invention may be carried out by acids, and the peptide bond formation may be carried out by a mixed-anhydride method, a carbodiimide-additive method, or an active-ester method.

As the amino acid corresponding to the compound represented by the formula (6), there may be mentioned 1,3-thiazolidine-4-carboxylic acid [H-Thz-OH] and 5,5-dimethyl-1,3-thiazolidine-4-carboxylic acid [H-Dtc-OH]. Any amino protective group which is removed under acidic condition may be used for the group represented by A¹ in the formula (6), but *tert*-butoxycarbonyl is preferred. Any lower alkyl group may be used for the group represented by R³ in the formula (7) from the synthetic point of view, but *tert*-butyl is preferred for HIV protease inhibitory activity.

As the condensation method of the step C, a mixed-anhydride method and an active-ester method are preferred. In the mixed-anhydride method, an acyl chloride (normally 1.0 - 1.5 eq) is added into a solution of the compound represented by the formula (6) in the presence of an organic amine (normally 1.0 - 1.5 eq) to form the mixed anhydride. As said acyl chloride, for example, isobutyl chloroformate, isopropyl chloroformate, and pivaloyl chloride may be used. As said organic amine, for example, triethylamine and *N*-methylmorpholine may be used. As the reaction solvent, for example, tetrahydrofuran [THF], dimethoxyethane, acetonitrile, ethyl acetate, *N,N*-dimethylformamide [DMF], or mixtures thereof may be used. A reaction temperature between - 20 and - 10 °C is preferred.

In the active-ester method, a carbodiimide (normally 1.0 - 1.5 eq) is added to a solution of the compound represented by formula (6) in the presence of a hydroxyl compound such as *N*-hydroxybenzotriazole [HOBt] or *N*-hydroxysuccinimide [HOSu] (normally 1.0 - 1.5 eq) to form the active ester. Said reaction may be carried out in the presence of an organic amine such as pyridine. As said carbodiimide, for example, *N,N'*-dicyclohexylcarbodiimide [DCC] or 1-ethyl-3-(3-*N,N*-dimethylaminopropyl) carbodiimide [EDC] may be used, and the economically cheaper DCC is more preferred. As the reaction solvent, for example, THF, dioxane, dichloromethane, chloroform, acetonitrile, DMF, and mixtures thereof may be used. A reaction temperature between 0 and 30 °C is preferred.

The mixed anhydride or the active ester obtained by the above operation is reacted with the amine represented by formula (7). The reaction mixture of said mixed anhydnde or the active ester may be used without isolation. Normally 1 - 5 equivalents of amine may be used, and excess amount of said amine, especially over 2 equivalents, is preferably used to raise the yield. A reaction temperature between - 20 and 30 °C is preferred and that between - 10 and 15 °C is more preferred. The compound represented by formula (8) is obtained by the usual work-up after completion of the reaction.

The deprotection of step D is easily performed under acidic conditions. As the acid, for example, hydrogen chloride, hydrogen bromide, trifluoroacetic acid, methanesulfonic acid, or toluenesulfonic acid (normally 2 - 20 eq) may be used. As the reaction solvent, for example, methanol, dioxane, dichloromethane, acetic acid, formic acid, acetonitrile, ethyl acetate, or mixtures thereof may be used. Said acid, its amount, and said solvent may be chosen according to the protective group represented by A¹. The compound represented by formula (9) is produced as the corresponding ammonium-type salt, and said salt may be neutralized with a *tert*-amine such as triethylamine or *N*-methylmorpholine in the solvent of the subsequent step D. The compound represented by the formula (9) may also be used after isolation by alkaline work-up.

As the condensation method of step E, a carbodiimide-additive method is preferred. To a solution or suspension of the compound represented by formula (9), the compound represented by formula (10) (normally 0.8 - 1.2 eq, more preferably 0.9 - 1.1 eq) and an additive of the carbodiimide-additive method (normally 0.2 - 1.2 eq, more preferably 0.8 - 1.1 eq) in a reaction solvent, a carbodiimide (normally 1.0 - 1.5 eq, more preferably 1.0 - 1.2 eq) are added and react. Any amino protective group which is removed under acidic conditions may be used for the group represented by A² in formula (10), but *tert*-butoxycarbonyl is preferred. As said additive, a hydroxyl compound such as HOBt, HOSu, N-hydroxy-5-norbornene-2,3-dicarboximide [HONb], or 3-hydroxy-4-oxo-3,4-dihydro-1,2,3-benzotriazine may be used, but HOBt and HOSu are more preferred. As the reaction solvent, for example, THF, dioxane, acetonitrile, chloroform, dichloromethane, DMF, or mixtures thereof may be used. As said carbodiimide, for example, DCC or EDC may be used, and the economically cheaper DCC is more preferred. A reaction temperature between 0 and 30 °C is preferred. The compound represented by formula (11) is obtained by the usual work-up after completion of the reaction. Purification, for example by silica-gel column chromatography, may be performed.

The deprotection step F is easily performed under acid conditions similarly to that in step D. As the acid, for example, hydrogen chloride, hydrogen bromide, trifluoroacetic acid, methanesulfonic acid, or toluenesulfonic acid (normally 2 - 20 eq) may be used. As the reaction solvent, for example, methanol, dioxane, dichloromethane, acetic acid, formic acid, acetonitrile, ethyl acetate, or mixtures thereof may be used. Said acid, its amount, and said solvent may be chosen according to the protective group represented by A². The compound represented by formula (4) is produced as the corresponding ammonium-type salt, and said salt may be neutralized with a *tert*-amine such as triethylamine or *N*-methylmorpholine in the solvent of the subsequent step F. The compound represented by formula (4) may also be used after isolation by alkaline work-up.

As the condensation method of step B, a carbodiimide-additive method or an active-ester method is preferred. In the carbodiimide-additive method, a carbodiimide (normally 1.0 - 1.5 eq, more preferably 1.0 - 1.2 eq) is added to a solution or suspension of the compound represented by formula (4), the compound represented by formula (5) (normally 1.0 - 1.2 eq) and an additive of the carbodiimide-additive method (normally 0.2 - 1.2 eq, more preferably 0.8 - 1.1 eq) in a reaction solvent. the amino acid corresponding to the compound represented by the formula (5), there may be mentioned - methylthioalanine [H-Mta-OH], methanesulfonylalanine [H-Msa-OH], asparagine [H-Asn-OH], valine [H-Val-OH], isoleucine [H-Ile-OH], and alanine [H-Ala-OH]. Any amino protective group which is removed under acidic conditions may be used for the group represented by A³ in the formula (5), but *tert*-butoxycarbonyl is preferred. As said additive, a hydroxyl compound such as HOBt, HOSu, HONb, or *p*-nitrophenol [HONp] may be used. As the reaction solvent, for example, THF, dioxane, acetonitrile, chloroform, dichloromethane, DMF, or mixtures thereof may be used. As said carbodiimide, for example, DCC or EDC may be used, and the economically cheaper DCC is more preferred. A reaction temperature between 0 and 30 °C is preferred. The compound represented by formula (12) is obtained by the usual work-up after completion of the reaction. Purification, for example by silica-gel column chromatography or recrystallization, may be performed.

In the active-ester method, a carbodiimide (normally 1.0 - 1.5 eq) is added to a solution of the compound represented by formula (5) in the presence of a hydroxyl compound such as HOBt, HOSu, HONb, or HONp (normally 1.0 - 1.5 eq) to form the active ester. Said reaction may be carried out in the presence of an organic amine such as pyridine. Said active ester is obtained by the usual work-up after the completion of the reaction. As said carbodiimide, for example, DCC or EDC may be used, and the economically cheaper DCC is more preferred. As the reaction solvent, for example, THF, dioxane, dichloromethane, chloroform, acetonitrile, DMF, or mixtures thereof may be used. A reaction temperature between 0 and 30 °C is preferred.

Said active ester is reacted with the compound represented by formula (4) in the presence of amine such as triethylamine or *N*-methylmorpholine (normally 1.0 - 1.5 eq). The presence of a hydroxyl compound such as HOBt or HOSu (normally 0.5 - 1.5 eq) is more preferred. As the reaction solvent, for example, THF, dioxane, dichloromethane, chloroform, acetonitrile, DMF, dimethylsulfoxide, or mixtures thereof may be used. A reaction temperature between 0 and 30 °C is preferred. The compound represented by formula (12) is obtained by the usual work-up after the completion of the reaction. Purification, for example by silica-gel column chromatography or recrystallization, may be performed.

The deprotection of step G is easily performed under acidic conditions similarly to that in step D. As the acid, for example, hydrogen chloride, hydrogen bromide, trifluoroacetic acid, methanesulfonic acid, or toluenesulfonic acid (normally 2 - 20 eq) may be used. As the reaction solvent, for example, methanol, dioxane, dichloromethane, acetic acid, formic acid, acetonitrile, ethyl acetate, or mixtures thereof may be used. Said acid, its amount, and said solvent may be chosen according to the protective group represented by A³. The compound represented by formula (2) is produced as the corresponding ammonium-type salt, and said salt may be neutralized with a *tert*-amine such as triethylamine or *N*-methylmorpholine in the solvent of the subsequent step A. The compound represented by formula (2) may also be used after isolation by alkaline work-up.

As the condensation method of step A, a carbodiimide-additive method is preferred. To a solution or suspension of the compound represented by formula (2), the compound represented by formula (3) (normally 1.0 - 1.2 eq), an additive of the carbodiimide-additive method (normally 0.2 - 1.2 eq, preferably 0.8-1.1 eq) in a reaction solvent, and carbodiimide (normally 1.0 - 1.5 eq, preferably 1.0-1.2 eq)) are added to react. As said compound represented by formula (3), there may be mentioned 1-naphtyloxyacetic acid [Noa-OH], 5-isoquinolyloxyacetic acid [Qoa-OH], or 3-Pyridyloxyacetic acid [Pyoa-OH]. As said additive, a hydroxyl compound such as HOBt, HOSu, HONb, or HONp may be used, however, HOBt and HOSu are more preferred. As the reaction solvent, for example, THF, dioxane, acetonitrile, chloroform, dichloromethane, DMF, or mixtures thereof may be used. As said carbodiimide, for example, DCC or EDC may be used, and the economically cheaper DCC is more preferred. A reaction temperature between 0 and 30 °C is preferred. The compound represented by formula (1) is obtained by the usual work-up after completion of the reaction. Purification, for example by silica-gel column chromatography or recrystallization, may be performed.

In the present invention, the compound represented by formula (1) may be obtained from the compound represented by the formula (4) by performing condensation step B', instead of carring out steps B,G and A.

As the condensation method of step B', for example, a carbodiimide-additive method is preferred. To a solution or suspension of the compound represented by formula (4), the compound represented by formula (5') wherein the amino protective group A⁴ represents an acyl group derived from the carboxylic acid represented by the formula (3) (normally 1.0 - 1.2 eq), an additive of the carbodiimide-additive method (normally 0.2 - 1.2 eq, more preferably 0.8 - 1.1 eq) in a reaction solvent, and a carbodiimide (normally 1.0 - 1.5 eq, more preferably 1.0 - 1.2 eq) are added and reacts. As said compound represented by formula (5'), there may be mentioned *N*-(5-isoquinolyloxyacetyl)methylthioalanine, *N*-(5-isoquinolyloxyacetyl)methanesulfonylalanine, *N*-(5-isoquinolyloxyacetyl)asparagine, *N*-(5-isoquinolyloxyacetyl)valine, *N*-(5-isoquinolyloxyacetyl)isoleucine, *N*-(5-isoquinolyloxyacetyl)alanine, *N*-(1-naphthoxyacetyl)methylthioalanine, *N*-(1-naphthoxyacetyl)methanesulfonylalanine, *N*-(1-naphthoxyacetyl)asparagine, *N*-(1-naphthoxyacetyl)valine, *N*-(1-naphthoxyacetyl)isoleucine, *N*-(1-naphthoxyacetyl)alanine, *N*-(3-pyridyloxyacetyl)methylthioalanine, *N*-(3-pyridyloxyacetyl)methanesulfonylalanine, *N*-(3-pyridyloxyacetyl)asparagine, *N*-(3-pyridyloxyacetyl)valine, *N*-(3-pyridyloxyacetyl)isoleucine, *N*-(3-pyridyloxyacetyl)alanine. As said additive, a hydroxyl compound such as HOBt, HOSu, HONb, or HONp may be used. As the reaction solvent, for example, THF, dioxane, acetonitrile, chloroform, dichloromethane, DMF, and mixtures thereof may be used. As said carbodiimide, for example, DCC or EDC may be used, and the economically cheaper DCC is more preferred. A reaction temperature between 0 and 30 °C is preferred. The compound represented by formula (1) is obtained by the usual work-up after completion of the reaction. Purification, for example by silica-gel column chromatography or recrystallization,may be performed.

The compound represented by formula (1) may be purified by crystallization of the corresponding acid-addition salt when Ar is a basic heteroaryl group. The compound represented by formula (1) is mixed with a protic acid in a solvent and the salt is crystallized upon standing or cooling. Recrystallization and/or desalting by alkaline work-up may also be performed. As said protic acid, there may be mentioned hydrogen chloride, acetic acid, propionic acid, butyric acid, valeric acid, pivalic acid, succinic acid, maleic acid, fumaric acid, malonic acid, glutaric acid, benzoic acid, salicylic acid, cinnamic acid, tartaric acid, citric acid, methanesulfonic acid, or toluenesulfonic acid. As said solvent, for example, ethanol, propanol, isopropanol, acetone, ethyl acetate, or mixtures thereof may be used.

In the process of the present invention, the compound represented by formula (1) may be stereospecifically obtained with little epimerization when optically active compounds represented by formulae (6), (10), (5) and (5') are used as the starting materials. Any stereo-isomer is used as said starting material, but the preferred configuration for HIV protease inhibitory activity is as follows: *R* (L) for compound represented by formula (6); (2*S*,3*S*) for the compound represented by the formula (10); *R* (L) for methylthioalanine and methanesulfonylalanine derivatives among the compound represented by formulae (5) and (5'); *S* (L) for asparagine, valine, isoleucine, and alanine derivatives among the compound represented by formulae (5) and (5').

Among said starting materials, the compound represented by formula (6) wherein A¹ represents *tert*-butoxycarbonyl may be produced by step I, and the compound represented by formula (5) wherein X represents methylthiomethyl and A³ represents rerr-butoxycarbonyl may be produced by step II.

In step I, formaldehyde (at least 1.0 eq, preferably 1.2 - 2.0 eq) is added to an aqueous solution of the compound represented by formula (13) or salt thereof to cyclize. As the compound represented by formula (13), for example, cysteine or penicillamine may be used. Formalin may preferably be used as the formaldehyde source. A reaction temperature between - 10 and 50 °C is preferred and that between 0 and 30 °C is more preferred. The completion of the cyclization reaction may be detected, for example, by TLC.

To the reaction mixture obtained above, di-*tert*-butyl dicarbonate (normally 1.0 - 1.5 eq) is added to react. The reaction is preferably carried out under neutral or slightly basic conditions by addition of a base such as triethylamine, *N*-methylmorpholine, sodium hydroxide, or potassium hydroxide. The presence of a polar organic solvent such as THF or dioxane is also preferred. A reaction temperature between - 10 and 50 °C is preferred and that between 0 and 30 °C is more preferred.

Acidification of the above reaction mixture gives the compound represented by formula (6) wherein A¹ represents *tert*-butoxycarbonyl. As the acid for said acidification, a water soluble organic acid such as citric acid or mineral acid such as hydrochloric acid may be used and hydrochloric acid is cheap and more preferable. Before the acidification, the organic solvent used may preferably be distilled off and the resultant aqueous solution may preferably be washed with an organic solvent such as toluene, ethyl acetate, or ether. The compound represented by formula (6) wherein A¹ represents *tert*-butoxycarbonyl may be isolated by crystallization from the aqueous mixture obtained above or extraction with an organic solvent such as toluene, ethyl acetate, or ether. The compound represented by formula (6) wherein A¹ represents *tert*-butoxycarbonyl may be obtained in optically active form without racemization from an optically active compound represented by formula (13).

In the step II, cysteine or its salt is neutralized by alkali such as sodium hydroxide and reacted with methyl halide (normally more than 1.0 eq, preferably 1.0 - 1.1 eq), such as methyl iodide, in an aqueous solvent. An organic solvent such as THF, dioxane, or ether may be preferably used as a co-solvent. A reaction temperature between - 10 and 50 °C is preferred and that between 0 and 30 °C is more preferred. The completion of the methylation reaction may be detected, for example, by TLC.

To the reaction mixture obtained above, di-*tert*-butyl dicarbonate (normally 1.0 - 1.5 eq) is added to react. The reaction is preferably carried out under neutral or slightly basic conditions by addition of a base such as triethylamine, *N*-methylmorpholine, sodium hydroxide, or potassium hydroxide. A presence of a polar organic solvent such as THF or dioxane is also preferred. The reaction temperature between - 10 and 50 °C is preferred and that between 0 and 30 °C is more preferred.

Acidification of the above reaction mixture gives the compound represented by formula (5) wherein X represents methylthiomethyl and A³ represents *tert*-butoxycarbonyl. As the acid for said acidification, a water soluble organic acid such as citric acid or mineral acid such as hydrochloric acid may be used and hydrochloric acid is cheap and more preferable. Before the acidification, the organic solvent used may preferably be distilled off and the resultant aqueous solution may preferably be washed with an organic solvent such as toluene, ethyl acetate, or ether. The compound represented by formula (5), wherein X represents methylthiomethyl and A³ represents *tert*-butoxycarbonyl, may be isolated by crystallization from the aqueous mixture obtained above or by extraction with organic solvent such as toluene, ethyl acetate, or ether. Said extract may be preferably washed with a solution of a reducing agent such as sodium bisulfite or sodium thiosulfate. The compound represented by the formula (5), wherein X represents methylthiomethyl and A³ represents *tert*-butoxycarbonyl may be obtained in an optically active form without racemization from optically active cysteine.

The present invention will be illustrated in more detail with reference to the following examples, which should not be construed to be limiting to the scope of the invention.

### Example 1

To a solution of 20.00 g (114 mmol) of L-cysteine hydrochloride monohydrate in 60 ml of water, 12 ml (1.4 eq) of 37% formalin containing 8% methanol was added and the resultant mixture was stirred for 6 h at room temperature. To this, 76 ml (2.0 eq) of 3 N NaOH aqueous solution and a solution of 27.4 g (1.1 eq) of di-*tert*-butyl dicarbonate in 60 ml of THF were added and the mixture was stirred for 2 h. After addition of 8 ml (0.2 eq) of 3 N NaOH aqueous solution, the reaction mixture was stirred overnight at room temperature, concentrated to remove THF under reduced pressure, washed with 80 ml of toluene, acidified by 23 ml of 6 N hydrochloric acid, and extracted with 160 ml of ethyl acetate. The organic extract was concentrated under reduced pressure, dried by toluene azeotrope, and crystallized in hexane to give 25.37 g (Y. 95.3%) of (*R*)-3-*tert*-butoxycarbonyl-1,3-thiazolidine-4-carboxylic acid [Boc-Thz-OH].
¹H NMR (CDCl₃): δ 1.48 (s, 9H), 3.31 (bs, 2H), 4.3 - 4.9 (m, 3H), 8.27 (b, 1H).

### Example 2

To a solution of 125.0 g (712 mmol) of L-cysteine hydrochloride monohydrate in 250 ml of water, 475 ml (2.0 eq) of 3 N NaOH aqueous solution and a solution of 106.1 g (1.05 eq) of iodomethane in 250 ml of THF were added successively under ice cooling. After stirring for 2 h at that temperature, 250 ml (1.05 eq) of 3 N NaOH aqueous solution and a solution of 170.9 g (1.1 eq) of di-*tert*-butyl dicarbonate in 250 ml of THF were added and the mixture was stirred for 3.5 h at room temperature. After addition of 48 ml (0.2 eq) of 3 N NaOH aqueous solution, the resultant mixture was stirred for 15 h at room temperature, concentrated to remove THF under reduced pressure, washed with 500 ml of toluene, acidified by 140 ml of 6 N hydrochloric acid, and extracted with 1000 ml of ethyl acetate. The organic extract was washed with 500 ml of 5% NaCl aqueous solution containing 0.5% NaHSO₃, concentrated under reduced pressure, dried by toluene azeotrope, and crystallized in hexane to give 159.7 g (Y. 95%) of (*R*)-2-(*N*-*tert*-butoxycarbonylamino)-3-methylthiopropanoic acid [Boc-Mta-OH].
¹H NMR (CDCl₃): δ 1.46 (s, 9H), 2.16 (s, 3H), 2.99 (m, 2H), 4.55 (m, 1H), 5.39 (m, 1H), 8.79 (b, 1H).

### Example 3

### [Process 1]

To a solution of 25.0 g (107 mmol) of (*R*)-3-*tert*-butoxycarbonyl-1,3-thiazolidine-4-carboxylic acid [Boc-Thz-OH: (*R*) configuration unless otherwise noted] in 200 ml of THF, 12.3 g (107 mmol) of *N*-hydroxysuccinimide [HOSu] and 24.3 g (118 mmol) of *N,N*'-dicyclohexylcarbodiimide [DCC] were added successively under ice cooling. After stirring for 1 h, 56.2 ml (535 mmol) of *tert*-butylamine was added under ice cooling and the reaction mixture was stirred for 1 h and filtered. The precipitates were washed with 200 ml of THF and the combined THF solution was concentrated under reduced pressure. The residue was dissolved in 200 ml of ethyl acetate, washed with 5% citric acid aqueous solution, and filtered. The organic solution was washed with 5% citric acid aqueous solution, 5% NaHCO₃ aqueous solution, and saturated NaCl aqueous solution successively, dried over Na₂SO₄, and concentrated under reduced pressure. The residue was crystallized by addition of 400 ml of hexane to give 22.8 g (Y. 74%) of Boc-Thz-NH-tBu.
¹H NMR (CDCl₃, 270 MHz): δ 1.35 (s, 9H), 1.49 (s, 9H), 3.20 (b, 1H), 3.36 (b, 1H), 4.35 (bd, 1H), 4.53 (b, 1H), 4.65 (d, 9.2 Hz, 1H), 5.96 (b, 1H).
HPLC: 19.5 min (column: YMC AM-302, 4.6 x 150; eluting solution A: 0.1% trifluoroaceetic acid aqueous solution; eluting solution B: acetonitrile; linear gradient: 100% A to 100% B for 30 min; flow rate: 1.0 ml/min).

### [Process 2]

To 6.49 g (22.5 mmol) of Boc-Thz-NH-tBu, 8.45 ml (67.6 mmol) of 8 M solution of methanesulfonic acid in acetonitrile was added under ice cooling. After stirring for 5 min under ice cooling and for 40 min at room temperature, 70 ml of dichloromethane and 100 ml of 1 N NaOH aqueous solution were added under ice cooling to extract the product. The resultant dichloromethane solution was washed with water, dried over Na₂SO₄, and concentrated under reduced pressure. The residue was crystallized by addition of 50 ml of hexane to give 3.50 g (Y. 83%) of H-Thz-NH-tBu.
¹H NMR (CDCl₃, 270 MHz): δ 1.35 (s, 9H), 2.37 (b, 1H), 3.08 (dd, 10.8 Hz, 7.6 Hz, 1H), 3.43 (dd, 10.8 Hz, 4.6 Hz, 1H), 3.95 (d, 9.8 Hz, 1H), 4.0 (m, 1H), 4.22 (d, 9.8 Hz, 1H), 6.88 (b, 1H).
HPLC: 10.1 min (condition: see process 1).

### [Process 3]

To a solution of 3.22 g (10.9 mmol) of (2*S*,3*S*)-3-*N*-*tert*-butoxycarbonylamino-2-hydroxy-4-phenylbutanoic acid [Boc-AHPBA-OH: (2*S*,3*S*) configuration unless otherwise noted] and 2.26 g (12.0 mmol) of H-Thz-NH-tBu in 30 ml of DMF, 1.48 g (9.7 mmol) of 1-hydroxybenzotriazol [HOBt] monohydrate and 2.70 g (13.1 mmol) of DCC were added successively under ice cooling. The reaction mixture was stirred at room temperature overnight, filtered to remove *N,N'*-dicyclohexylurea, and concentrated under reduced pressure. To the residue, 50 ml of ethyl acetate and 50 ml of 5% NaHCO₃ aqueous solution were added and the resultant mixture was stirred for 3 h and filtered. The organic layer was washed with 5% NaHCO₃ aqueous solution, 5% citric acid aqueous solution, and saturated NaCl aqueous solution successively, dried over Na₂SO₄, and concentrated under reduced pressure. The residue was purified by silica-gel column chromatography (chloroform - methanol) to give 5.07 g (Y. 100%) of Boc-AHPBA-Thz-NH-tBu.
HPLC: 21.8 min (condition: see process 1).

### [Process 4]

To 5.07 g (10.9 mmol) of Boc-AHPBA-Thz-NH-tBu, 27.3 ml (109 mmol) of 4 M HCl solution in dioxane was added under ice cooling, and the reaction mixture was stirred for 2 h, concentrated under reduced pressure, and dissolved in 50 ml of DMF. To this, 1.52 ml (10.9 mmol) of triethylamine, 2.56 g (10.9 mmol) of (R)-2-*N-tert*-butoxycarbonylamino-3-methylthiopropanoic acid [Boc-Mta-OH: (*R*) configuration unless otherwise noted], 1.67 g (10.9 mmol) of HOBt monohydrate and 2.47 g (12.0 mmol) of DCC were added successively under ice cooling. The reaction mixture was stirred at room temperature overnight, filtered to remove *N,N*'-dicyclohexylurea, and concentrated under reduced pressure. To the residue, 100 ml of 5% citric acid aqueous solution was added and precipitates formed were collected by filtration. The precipitates were washed with 5% citric acid aqueous solution, 5% NaHCO₃ aqueous solution, and water successively, dried under reduced pressure, dissolved in 200 ml of hot THF, and filtered. The filtrate was concentrated under reduced pressure and residue was crystallized by addition of ether to give 5.15 g (Y. 81%) of Boc-Mta-AHPBA-Thz-NH-tBu.
HPLC: 22.0 min (condition: see process 1).

### [Process 5]

To 2.00 g (3.44 mmol) of Boc-Mta-AHPBA-Thz-NH-tBu, 17.2 ml (68.7 mmol) of 4 M HCl solution in dioxane was added under ice cooling, and the reaction mixture was stirred for 2 h, concentrated under reduced pressure, and dissolved in 20 ml of DMF. To this, 0.48 ml (3.44 mmol) of triethylamine, 0.91 g (4.47 mmol) of 5-isoquinolyloxyacetic acid [Qoa-OH], 0.53 g (3.44 mmol) of HOBt monohydrate and 0.92 g (4.47 mmol) of DCC were added successively under ice cooling. The reaction mixture was stirred at room temperature overnight, filtered to remove *N,N'*-dicyclohexylurea, and concentrated under reduced pressure. To the residue, 100 ml of 5% NaHCO₃ aqueous solution was added and extracted with 100 ml of ethyl acetate. The organic layer was washed with 5% NaHCO₃ aqueous solution and saturated NaCl aqueous solution succesively, dried over Na₂SO₄, and concentrated under reduced pressure. The residue was crystallized by addition of hexane to give 2.24 g (Y. 95%) of Qoa-Mta-AHPBA-Thz-NH-tBu.
HPLC: 14.0 min (column: YMC AM-302, 4.6 x 150; eluting solution A: 0.1% trifluoroaceetic acid aqueous solution; eluting solution B: acetonitrile; linear gradient: 20% B to 80% B for 30 min; flow rate: 0.7 ml/min).

### [Process 6]

To a solution of 101 mg (147 mmol) of Qoa-Mta-AHPBA-Thz-NH-tBu in 700 ml of ethanol, 300 ml (150 mmol) of 0.5 M solution of acetic acid in ethanol was added. After standing at room temperature for 3 days, the corresponding acetate salt was obtained by centrifugation.

### Example 4

To a solution of 5.0 g (21.4 mmol) of Boc-Thz-OH in 30 ml of THF, 3.28 g (21.4 mmol) of HOBt monohydrate and 4.86 g (23.6 mmol) of DCC were added successively under ice cooling. After stirring for 1.5 h, a solution of 6.8 ml (65 mmol) of *tert*-butylamine in 20 ml of THF was added under ice cooling and the reaction mixture was stirred for 2 h and filtered. To the filtrate, 50 ml of toluene and 50 ml of 5% citric acid aqueous solution were added, and the resultant mixture was stirred and filtered. The organic layer was washed with 5% citric acid aqueous solution, 5% NaHCO₃ aqueous solution, and saturated NaCl aqueous solution successively, dried over Na₂SO₄, and concentrated under reduced pressure. The residue was crystallized by addition of 100 ml of hexane to give 4.92 g (Y. 80%) of Boc-Thz-NH-tBu which was converted to Qoa-Mta-AHPBA-Thz-NH-tBu by a method similar to processes 2 - 5 of Example 3.

### Example 5

To a solution of 3.0 g (12.9 mmol) of Boc-Thz-OH and 2.15 ml (15.5 mmol) of triethylamine in 25 ml of DMF, 2.02 ml (15.5 mmol) of isobutyl chloroformate was added dropwise at - 15 °C. After stirring for 10 min at that temperature, 4.06 ml (38.7 mmol) of *tert*-butylamine was added. The reaction mixture was stirred for 10 min at that temperature and 100 min under ice cooling, concentrated under reduced pressure, diluted with 50 ml of ethyl acetate, washed with 5% NaHCO₃ aqueous solution, 5% citric acid aqueous solution, and saturated NaCl aqueous solution successively, dried over Na₂SO₄, and evaporated under reduced pressure to give 3.13 g (Y. 84%) of Boc-Thz-NH-tBu which was converted to Qoa-Mta-AHPBA-Thz-NH-tBu by a method similar to processes 2 - 5 of Example 3.

### Example 6

To a solution of 3.0 g (12.9 mmol) of Boc-Thz-OH and 1.97 ml (14.2 mmol) of triethylamine in 30 ml of DMF, 1.75 ml (14.2 mmol) of pivaloyl chloride was added dropwise at - 20 °C. After stirring for 15 min at that temperature, 4.06 ml (38.7 mmol) of *tert*-butylamine was added. The reaction mixture was allowed to warm to room temperature during 4 h with stirring. A method similar to that of Example 5 gave 3.53 g (Y. 95%) of Boc-Thz-NH-tBu which was converted to Qoa-Mta-AHPBA-Thz-NH-tBu by a method similar to processes 2 - 5 of Example 3.

### Example 7

To 3.25 g (11.3 mmol) of Boc-Thz-NH-tBu, 15 ml (195 mmol) of trifluoroacetic acid was added under ice cooling and the resultant mixture was stirred at that temperature for 30 min and evaporated. The residue was washed with hexane by decantation and partitioned between chloroform and 1 N NaOH aqueous solution. The organic layer was dried over Na₂SO₄ and evaporated under reduced pressure to give 2.00 g (Y. 94%) of H-Thz-NH-tBu which was converted to Qoa-Mta-AHPBA-Thz-NH-tBu by a method similar to processes 3 - 5 of Example 3.

### Example 8

To a solution of 5.00 g (17.4 mmol) of Boc-Thz-NH-tBu in 23.7 ml of acetonitrile-dichloromethane (1:6), 3.39 ml (52.1 mmol) of methanesulfonic acid was added. After stirring at room temperature for 1.5 h, 30 ml of dichloromethane and 26 ml (52 mmol) of 2 N NaOH aqueous solution were added under ice cooling to extract the product. The organic layer was washed with 5% NaHCO₃ aqueous solution and saturated NaCI aqueous solution successively, dried over Na₂SO₄, and evaporated under reduced pressure. The residue was triturated in 50 ml of hexane to give 2.86 g (Y. 88%) of H-Thz-NH-tBu which was converted to Qoa-Mta-AHPBA-Thz-NH-tBu by a method similar to processes 3 - 5 of Example 3.

### Example 9

To 1.96 g (6.8 mmol) of Boc-Thz-NH-tBu, 17 ml (68 mmol) of 4 M HCI solution in dioxane was added under ice cooling. After stirring for 30 min, the reaction mixture was concentrated under reduced pressure and the residue was dissolved in 60 ml of DMF. To the resultant solution, 0.95 ml (6.8 mmol) of triethylamine, 1.80 g (6.8 mmol) of Boc-AHPBA-OH, 1.00 g (7.5 mmol) of HOBt monohydrate, and 1.56 g (8.2 mmol) of 1-ethyl-3-(3-*N,N*-dimethylaminopropyl)carbodiimide [EDC] hydrochloride were added successively under ice cooling. The reaction mixture was allowed to warm to room temperature and stirred overnight, and concentrated under reduced pressure. The residue was dissolved in ethyl acetate, washed with 5% citric acid aqueous solution, 5% NaHCO₃ aqueous solution, and saturated NaCI aqueous solution succesively, dried over Na₂SO₄, and evaporated under reduced pressure to give 2.69 g (Y. 87%) of Boc-AHPBA-Thz-NH-tBu which was converted to Qoa-Mta-AHPBA-Thz-NH-tBu by a method similar to processes 4 - 5 of Example 3.

### Example 10

### [Process 1]

To 2.00 g (4.3 mmol) of Boc-AHPBA-Thz-NH-tBu, 4.30 ml (17.2 mmol) of 4 M methanesulfonic acid solution in chloroform-acetonitrile (1:1) was added under ice cooling. After stirring for 30 min, the reaction mixture was diluted with 10 ml of DMF. To the resultant solution, 2.51 ml (18 mmol) of triethylamine, 1.01 g (4.30 mmol) of Boc-Mta-OH, 0.658 g (4.30 mmol) of HOBt monohydrate, and 0.907 g (4.73 mmol) of EDC hydrochloride were added successively under ice cooling. The reaction mixture was stirred at room temperature overnight and diluted with 5% NaHCO₃ aqueous solution. The precipitates formed were collected by filtration and washed with water. A subsequent re-precipitation from DMF-DMSO-water gave 1.90 g (Y. 76%) of Boc-Mta-AHPBA-Thz-NH-tBu.

### [Process 2]

To a suspension of 100 mg (0.17 mmol) of Boc-Mta-AHPBA-Thz-NH-tBu in 0.2 ml of acetonitrile-dichloromethane (1:1), 67 µl (1.03 mmol) of methanesulfonic acid was added. After stirring for 30 min, the reaction mixture was diluted with 0.3 ml of DMF. To the resultant mixture, 14.3 µl (1.03 mmol) of triethylamine, 35 mg (0.17 mmol) of Qoa-OH, 23 mg (0.17 mmol) of HOBt monohydrate, and 40 mg (0.21 mmol) of EDC hydrochloride were added successively under ice cooling. The reaction mixture was stirred at room temperature overnight, diluted with 5% NaHCO₃ aqueous solution, and extracted with ethyl acetate. The organic layer was evaporated under reduced pressure and the residue was crystalized by addition of hexane to give 94 mg (Y. 80%) of Qoa-Mta-AHPBA-Thz-NH-tBu.

### Example 11

### [Process 1]

To a solution of 20.0 g (85.1 mmol) of Boc-Mta-OH, 9.79 g (85.1 mmol) of HOSu, and 0.67 ml (8.5 mmol) of pyridine in 100 ml of THF, 21.0 g (102 mmol) of DCC was added under ice cooling. After stirring at room temperature for 15 h, the reaction mixture was filtered to remove *N,N*'-dicyclohexylurea, and concentrated under reduced pressure. To the residue, 200 ml of 2-propanol was added to crystallize 17.6 g (Y. 62%) of Boc-Mta-OSu.
¹H NMR (CDCl₃, 270 MHz): δ 1.47 (s, 9H), 2.19 (s, 3H), 2.85 (s, 4H), 3.02 (ABX, 14.2 Hz, 6.2 Hz, 1H), 3.12 (ABX, 14.2 Hz, 5.1 Hz, 1H), 4.88 (m, 1H), 5.34 (m, 1H).

### [Process 2]

To 8.64 g (18.6 mmol) of Boc-AHPBA-Thz-NH-tBu, 46.5 ml (186 mmol) of 4 M HCI solution in dioxane was added, and the reaction mixture was stirred for 1.5 h, concentrated under reduced pressure, and dissolved in 50 ml of DMF. To this, 2.58 ml (18.6 mmol) of triethylamine and 7.40 g (22.3 mmol) of Boc-Mta-OSu were added successively under ice cooling. After stirring for 1.5 h, 0.83 ml (5.9 mmol) of triethylamine was added and stirring was continued for 1.5 h. The reaction mixture was filtered and the filtrate was concentrated under reduced pressure. To the residue, 200 ml of 5% NaHCO₃ aqueous solution and 20 ml of ether were added and precipitates formed were collected by filtration. The precipitates were washed with 5% NaHCO₃ aqueous solution, 5% citric acid aqueous solution, water, 50% aqueous acetone, and hexane successively, dried under reduced pressure to give 9.00 g (Y. 83%) of Boc-Mta-AHPBA-Thz-NH-tBu which was converted to Qoa-Mta-AHPBA-Thz-NH-tBu by a method similar to process 5 of Example 3.

### Example 12

To a solution of 3.00 g (12.8 mmol).of Boc-Mta-OH and 1.78 g (12.8 mmol) of *p*-nitrophenol [HONp] in 16 ml of THF-chloroform (1:1), 2.89 g (14.1 mmol) of DCC was added under ice cooling. After stirring for 1h, the reaction mixture was filtered to remove *N,N*'-dicyclohexylurea, and concentrated under reduced pressure.

To the residue, 2-propanol is added to crystallize Boc-Mta-ONp which is used -instead of Boc-Mta-OSu in a method- similar to process 2 of Example 11 and converted to Qoa-Mta-AHPB A-Thz-NH-tBu.

### Example 13

To 1.50 g (2.58 mmol) of Boc-Mta-AHPBA-Thz-NH-tBu, 13 ml (52 mmol) of 4 M HCl solution in dioxane was added under ice cooling, and the reaction mixture was stirred for 80 min, concentrated under reduced pressure, and dissolved in 15 ml of DMF. To this, 0.36 ml (2.58 mmol) of triethylamine, 0.57 g (2.84 mmol) of 1-naphthoxyacetic acid [Noa-OH], 0.40 g (2.58 mmol) of HOBt monohydrate and 0.58 g (2.83 mmol) of DCC were added successively under ice cooling. The reaction mixture was stirred at room temperature overnight and treated similarly to process 5 of Example 3 to give 1.40 g (Y. 81%) of Noa-Mta-AHPBA-Thz-NH-tBu.

### Example 14

### [Process 1]

To a solution of 5.00 g (19.1 mmol) of (*R*)-3-*tert*-butoxycarbonyl-5,5-dimethyl-1,3-thiazolidine-4-carboxylic acid [Boc-Dtc-OH: (*R*) configuration unless otherwise noted] in 40 ml of THF, 2.20 g (19.1 mmol) of HOSu and 4.30 g (21.0 mmol) of DCC were added successively under ice cooling. After stirring for 2 h at room temperature, 10 ml (21 mmol) of *tert*-butylamine was added under ice cooling and the reaction mixture was treated similarly to process 1 of Example 3 to give 4.20 g (Y. 70%) of Boc-Dtc-NH-tBu.
¹H NMR (CDCl₃, 270 MHz): δ 1.35 (s, 9H), 1.42 (s, 3H), 1.46 (s, 9H), 1.55 (s, 3H), 3.90 (bs, 1H), 4.62 (s, 2H), 5.76 (b, 1H).

### [Process 2]

To 15.0 g (47.4 mmol) of Boc-Dtc-NH-tBu, 17.6 ml (141 mmol) of 8 M solution of methanesulfonic acid in acetonitrile was added. After stirring for 1 h, 90 ml of ether and 49 ml of 4 N NaOH aqueous solution were added under ice cooling to extract the product. The aqueous layer was extracted with 100 ml of ether and the combined ether solution was washed with 5% NaHCO₃ aqueous solution and saturated NaCl aqueous solution successively, dried over Na₂SO₄, and evaporated under reduced pressure. The residue was crystallized by addition of 15 ml of hexane to give 9.9 g (Y. 97%) of H-Dtc-NH-tBu.
¹H NMR (CDCl₃, 270 MHz): δ 1.32 (s, 3H), 1.37 (s, 9H), 1.68 (s, 3H), 2.8 (b, 1H), 3.32 (s, 1H), 4.17 and 4.26 (AB, 9.7 Hz, 2H), 6.31 (b, 1H).

### [Process 3]

To a solution of 4.09 g (13.8 mmol) of Boc-AHPBA-OH and 3.00 g (13.8 mmol) of H-Dtc-NH-tBu in 15 ml of DMF-chloroform (1:1), 2.12 g (13.8 mmol) of HOBt monohydrate and 3.14 g (15.2 mmol) of DCC were added successively under ice cooling. The reaction mixture was stirred at room temperature for 2 days and concentrated under reduced pressure. To the residue, 40 ml of ether and 40 ml of 5% citric acid aqueous solution were added and the resultant mixture was stirred for 15 min and filtered. The organic layer was washed with 5% citric acid aqueous solution, filtered, washed with 5% NaHCO₃ aqueous solution and saturated NaCl aqueous solution successively, dned over Na₂SO₄, and evaporated under reduced pressure to give 6.38 g (Y. 93%) of Boc-AHPBA-Dtc-NH-tBu.

### [Process 4]

To 3.00 g (6.07 mmol) of Boc-AHPBA-Dtc-NH-tBu, 15.2 ml (60.8 mmol) of 4 M HCl solution in dioxane was added under ice cooling, and the reaction mixture was stirred for 1 h, concentrated under reduced pressure, and dissolved in 15 ml of DMF-chloroform (1:1). To this, 0.845 ml (6.07 mmol) of triethylamine, 1.43 g (6.08 mmol) of Boc-Mta-OH, 0.93 g (6.07 mmol) of HOBt monohydrate and 1.38 g (6.7 mmol) of DCC were added successively under ice cooling. The reaction mixture was allowed to warm to room temperature, stirred overnight, and concentrated under reduced pressure. To the residue, 40 ml of ether and 40 ml of 5% citric acid aqueous solution were added and the resultant mixture was stirred for 15 min and filtered. The organic layer was washed with 5% citric acid aqueous solution, filtered, washed with 5% NaHCO₃ aqueous solution and saturated NaCl aqueous solution successively, dried over Na₂SO₄, and evaporated under reduced pressure to give 3.43 g (Y. 92%) of Boc-Mta-AHPBA-Dtc-NH-tBu.

### [Process 5]

To 1.90 g (3.11 mmol) of Boc-Mta-AHPBA-Dtc-NH-tBu, 7.7 ml (31 mmol) of 4 M HCI solution in dioxane was added under ice cooling, and the reaction mixture was stirred for 1 h, concentrated under reduced pressure, and dissolved in 15 ml of DMF. To this, 0.475 ml (3.4 mmol) of triethylamine, 0.82 g (4.0 mmol) of 5Qoa-OH, 0.48 g (3.11 mmol) of HOBt monohydrate and 0.83 g (4.0 mmol) of DCC were added successively under ice cooling. The reaction mixture was allowed to warm to room temperature, stirred overnight, and concentrated under reduced pressure. To the residue, 40 ml of ethyl acetate and 40 ml of 5% NaHCO₃ aqueous solution were added and the resultant mixture was stirred for 15 min and filtered. The organic layer was washed with 5% NaHCO₃ aqueous solution and saturated NaCl aqueous solution successively, dried over Na₂SO₄, and evaporated under reduced pressure. The residue was crystallized from ethyl acetate-hexane to give 1.60 g (Y. 74%) of Qoa-Mta-AHPBA-Dtc-NH-tBu.

### Example 15

To a solution of 5.15 g (34.5 mmol) of L-penicillamine in 50 ml of water, 3.4 ml (1.3 eq) of 37% formalin containing 8% methanol was added and the resultant mixture was stirred overnight at room temperature. To this, 7.2 ml (1.5 eq) of triethylamine and a solution of 9.07 g (1.2 eq) of di-*tert*-butyl dicarbonate in 50 ml of THF were added and the mixture was stirred for 8 h at room temperature, washed with ether, acidified (pH 3) by citric acid, and extracted with ethyl acetate. The organic extract was washed with 5% citric acid aqueous solution and saturated NaCl aqueous solution successively, dried over Na₂SO₄, evaporated under reduced pressure, and crystallized in hexane to give 8.39 g (Y. 92%) of (*R*)-3-*ter*t-butoxycarbonyl-5,5-dimethyl-1,3-thiazolidine-4-carboxylic acid [Boc-Dtc-OH].
¹H NMR (CDCl₃): δ 1.44 (s, 3H), 1.49 (s, 9H), 1.61 (s, 3H), 4.22 and 4.35 (b, 1H), 4.63 and 4.69 (b, 2H), 7.7 (b, 1H)

### Example 16

### [Process 1]

To a solution of 3.48 g (11.8 mmol) of Boc-AHPBA-OH and 2.55 g (11.8 mmol) of H-Dtc-NH-tBu in 13 ml of DMF-dichloromethane (1:1), 1.81 g (11.8 mmol) of HOBt monohydrate and 2.43 g (11.8 mmol) of DCC were added successively under ice cooling. The reaction mixture was stirred for 20 h at room temperature and concentrated under reduced pressure. To the residue, 30 ml of ether and 30 ml of 5% citric acid aqueous solution were added and the resultant mixture was stirred and filtered. The organic layer was washed with 5% citric acid aqueous solution, filtered, washed with 5% NaHCO₃ aqueous solution and saturated NaCl aqueous solution successively, dried over Na₂SO₄, and evaporated under reduced pressure to give 5.02 g (Y. 89%) of Boc-AHPBA-Dtc-NH-tBu.

### [Process 2]

To 3.95 g (8.0 mmol) of Boc-AHPBA-Dtc-NH-tBu, 20 ml (80 mmol) of 4 M HCl solution in dioxane was added, and the reaction mixture was stirred for 30 min at room temperature, concentrated under reduced pressure, and dissolved in 50 ml of DMF. To this, 1.1 ml (8.0 mmol) of triethylamine, 4.24 g (12.0 mmol) of *p*-nitrophenyl (*S)*-2-*tert*-butoxycarbonylamino-3-carbamoylpropanoate [Boc-Asn-ONp: (*S*) configuration unless otherwise noted], and 1.3 ml (12.0 mmol) of *N*-methylmorpholine were added successively under ice cooling. After stirring for 1 h at room temperature, 1.84 g (12.0 mmol) of HOBt monohydrate was added and stirring was continued for 14 h. The reaction mixture was concentrated under reduced pressure and partitioned between 100 ml of ethyl acetate and 50 ml of 5% NaHCO₃ aqueous solution. The aqueous layer was extracted with 50 ml of ethyl acetate and the combined organic solution was washed with 5% NaHCO₃ aqueous solution, 5% citric acid aqueous solution, and saturated NaCl aqueous solution successively, dried over Na₂SO₄, and evaporated under reduced pressure to give 5.20g of crude Boc-Asn-AHPBA-Dtc-NH-tBu. To 2.60 g of the crude product, 10 ml (40 mmol) of 4 M HCl solution in dioxane was added, and the mixture was stirred for 1 h at room temperature, and evaporated under reduced pressure. The residue was triturated in ether to give 1.76 g (Y. 81%) of H-Asn-AHPBA-Dtc-NH-tBu hydrochloride.

### [Process 3]

To a solution of 1.45 g (2.7 mmol) of H-Asn-AHPBA-Dtc-NH-tBu hydrochloride in 20 ml of DMF, 0.37 ml (2.7 mmol) of triethylamine, 0.59 g (2.9 mmol) of 1-naphthoxyacetic acid [Noa-OH], 0.41 g (2.7 mmol) of HOBt monohydrate and 0.60 g (2.9 mmol) of DCC were added successively under ice cooling. The reaction mixture was stirred at room temperature overnight, filtered to remove insoluble matter, and partitioned between 50 ml of ethyl acetate and 20 ml of 5% citric acid aqueous solution. The aqueous layer was extracted with 30 ml of ethyl acetate and the combined organic solution was washed with 5% citric acid aqueous solution, 5% NaHCO₃ aqueous solution, and saturated NaCl aqueous solution successively, dried over Na₂SO₄, evaporated under reduced pressure, and purified by silica-gel column chromatography (chloroform-methanol) to give 1.10 g (Y. 59%) of Noa-Asn-AHPBA-Dtc-NH-tBu.

### Example 17

### [Process 1]

To a suspension of 2.03 g (10 mmol) of Qoa-OH and 1.35 g (10 mmol) of HOBt in 10 ml of DMF, a solution of 2.27 g (11 mmol) of DCC in 10 ml of DMF was added under ice cooling and the resultant mixture was stirred for 1 h. To this, a DMF solution of (*R*)-methylthioalanine methyl ester [H-Mta-OCH₃], which was prepared from 1.86 g (10 mmol) of the corresponding hydrochlonde by neutralization with 1.39 ml (10 mmol) of triethylamine in 15 ml of DMF, was added under ice cooling and the reaction mixture was allowed to warm to room temperature, stirred overnight, and diluted with 50 ml of ethyl acetate. To this, 70 ml of 10% citric acid aqueous solution was added dropwise under ice cooling and the mixture was stirred for 20 min at room temperature and filtered. To the filtrate, 50 ml of 10% NaHCO₃ aqueous solution was added and the aqueous layer was extracted with ethyl acetate. The combined organic solution was washed with 5% NaHCO₃ aqueous solution and 5% NaCl aqueous solution succesively, treated with 0.2 g of activated carbon, and evaporated under reduced pressure. The residue was crystallized by addition of hexane to give 2.28 g (Y. 68%) of Qoa-Mta-OCH₃.

### [Process 2]

To a solution of 1.34 g (4.0 mmol) of Qoa-Mta-OCH3 in 20 ml of methanol, 1.0 ml (4.0 mmol) of 4 N NaOH aqueous solution was added under ice cooling. After stirring for 3.5 h, the reaction mixture was neutralized by 0.23 ml (4.0 mmol) of acetic acid and evaporated under reduced pressure. The residue was dissolved in 40 ml of 0.1 N NaOH aqueous solution, filtered to remove insoluble matter, and washed with 20 ml of dichloromethane. The aqueous solution was neutralized with 1 N hydrochloric acid to adjust to pH 7 and the precipitates form were collected by filtration, washed with 10 ml of cold water, and dried under reduced pressure to give 0.89 g (Y. 70%) of Qoa-Mta-OH.

### [Process 3]

To a solution of 15.8 g (34 mmol) of Boc-AHPBA-Thz-NH-tBu, which was prepared by a method similar to process 1-3 of Example 3, in 40 ml of dichloromethane, 85 ml (340 mmol) of 4 M HCl solution in dioxane was added. Aftert stirring for 2 h, the reaction mixture was diluted with 240 ml of water and filtered. The aqueous layer of the filtrate was diluted with 80 ml of methanol, neutralized by portionwise addition of 34 g of NaHCO₃, and stirred overnight. The precipitates formed were collected by filtration, washed with 80 ml of water-methanol (1:1), and dried under reduced pressure to give 8.12 g (Y. 65%) of H-AHPBA-Thz-NH-tBu.

### [Process 4]

To a solution of 1.31 g (4.1 mmol) of Qoa-Mta-OH, 1.50 g (4.1 mmol) of H-AHPBA-Thz-NH-tBu, and 0.55 g (4.1 mmol) of HOBt in 38 ml of DMF, a solution of 0.93 g (4.5 mmol) of DCC in 3 ml of DMF was added under ice cooling and the reaction mixture was allowed to warm to room temperature, stirred overnight, and diluted with 30 ml of ethyl acetate. To this, 30 ml of 10% citric acid aqueous solution was added dropwise under ice cooling and the mixture was stirred for 1 h at room temperature and filtered. To the filtrate, 20 ml of 5% NaHCO₃ aqueous solution was added and the aqueous layer was extracted with 30 ml of ethyl acetate. The combined organic solution was washed with 5% K₂CO₃ aqueous solution and 5% NaCl aqueous solution successively, and evaporated under reduced pressure. The residue was purified by silica-gel column chromatography (dichloromethane-ethanol) -ethanol) to give 2.30 g (Y. 84%) of Qoa-Mta-AHPBA-Thz-NH-tBu.

### Example 18

### [Process 1]

To a solution of 1.00 g (2.74 mmol) of H-AHPBA-Thz-NH-tBu, 0.60 g (2.74 mmol) of Boc-Val-OH, and 0.37 g (2.74 mmol) of HOBt in 15 ml of DMF, 0.58 g (3.01 mmol) of EDC hydrochloride was added under ice cooling. After stirring overnight at room temperature, the reaction mixture was diluted with 90 ml of 5% citric acid aqueous solution. The precipitates formed were collected by filtration, washed with 3% K₂CO₃ aqueous solution and hot methanol successively, and dried under reduced pressure to give 0.92 g (Y. 60 %) of Boc-Val-AHPBA-Thz-NH-tBu.

### [Process 2]

To 0.92 g (1.64 mmol) of Boc-Val-AHPBA-Thz-NH-tBu, 4.1 ml (16 mmol) of 4 M HCI solution in dioxane was added, and the reaction mixture was stirred for 1 h at room temperature, concentrated under reduced pressure, and diluted with ether. The precipitates formed were collected by filtration, dried under reduced pressure, and dissolved in 10 ml of DMF. To this, 0.23 ml (1.64 mmol) of triethylamine, 0.40 g (1.97 mmol) of Qoa-OH, 0.30 g (1.97 mmol) of HOBt, and 0.38 g (1.97 mmol) of EDC hydrochloride were added successively under ice cooling and the reaction mixture was stirred overnight at room temperature. After evaporation under reduced pressure, the residue was partitioned between ethyl acetate and of 5% NaHCO₃ aqueous solution. The organic solution was washed with saturated NaCl aqueous solution, dried over Na₂SO₄, evaporated under reduced pressure, and purified by silica-gel column chromatography to give 0.65 g (Y. 60%) of Qoa-Val-AHPBA-Thz-NH-tBu.

### Example 19

The a method similar to process 2 of Example 18 may give Pyoa-Val-AHPBA-Thz-NH-tBu by use of 3-pyridyloxyacetic acid [Pyoa-OH] instead of Qoa-OH.

## Claims

1. A process for producing peptide derivative (1) or salt thereof 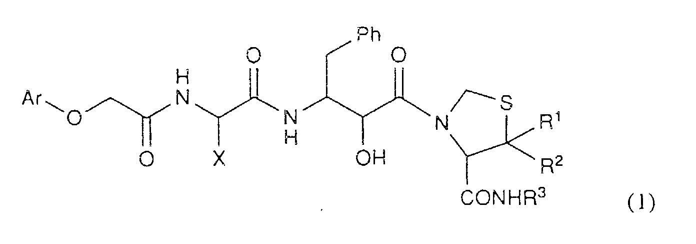 which comprises condensation of amino amide of formula (9) 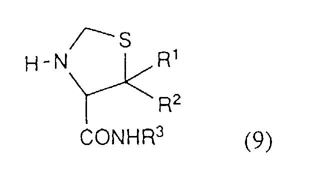 with protected amino acid of formula (10) 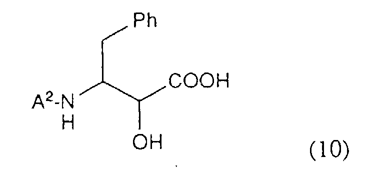 by carbodiimide additive method to give protected peptide of formula (11) 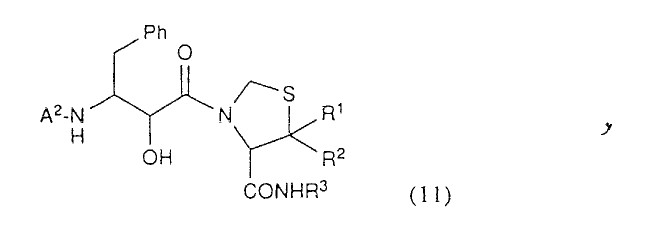 deprotection of said protected peptide to give peptide of formula (4) 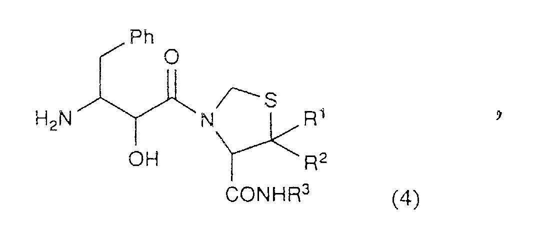 condensation of said peptide (4) with protected amino acid of formula (5) 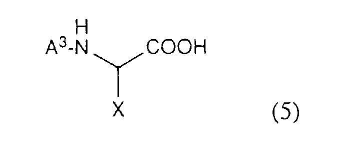 to give protected peptide of formula (12) 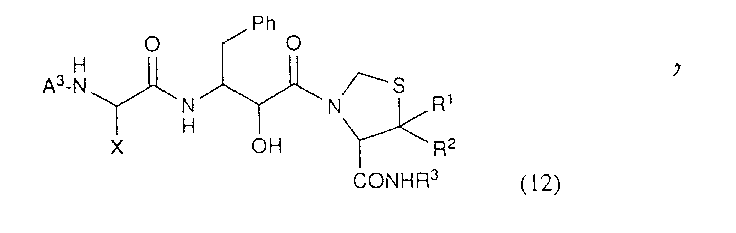 deprotection of protected peptide (12) to give peptide of formula (2) 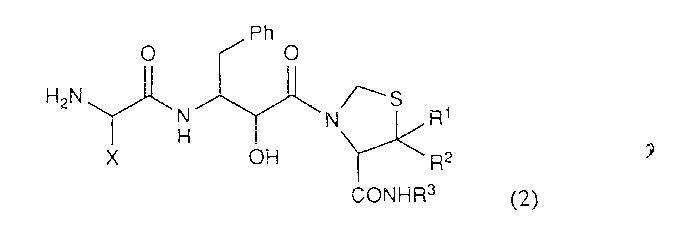 and condensation of peptide (2) with carboxylic acid of formula (3) 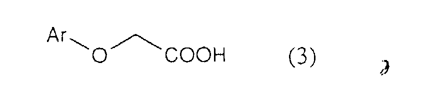 wherein A² and A³ are amino-protective groups, R¹ and R² are selected from C₁-C₆ alkyl groups and a hydrogen atom, R³ is a C₁-C₆ alkyl group, X is a methylthiomethyl, methanesulfonylmethyl, carbamoylmethyl or C₁-C₆ alkyl group and Ar is an aryl or heteroaryl group.

2. A process for producing peptide derivative (1) or salt thereof 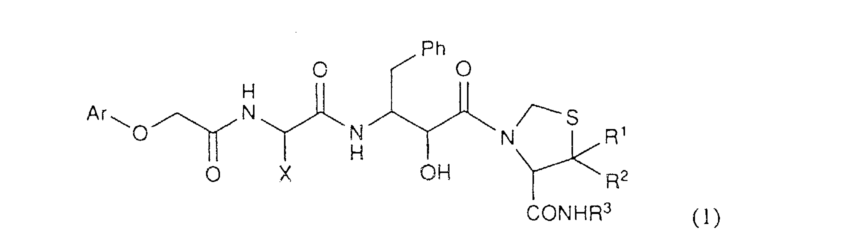 which comprises condensation of amino amide of formula (9) 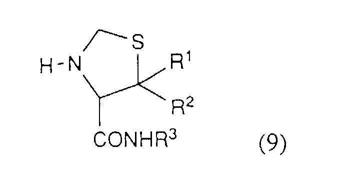 with protected amino acid of formula (10) 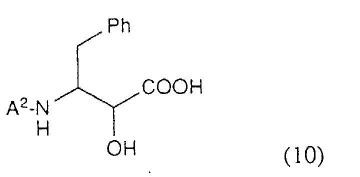 by carbodiimide-additive method to give protected peptide of formula (11) 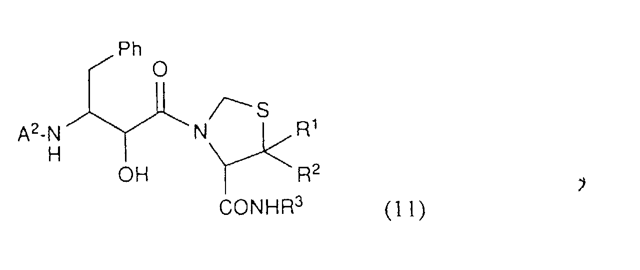 deprotection of said protected peptide to give peptide of formula (4) 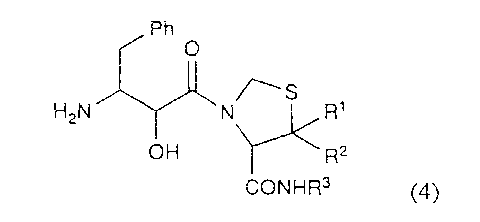 and condensation of peptide (4) with protected amino acid of formula (5') 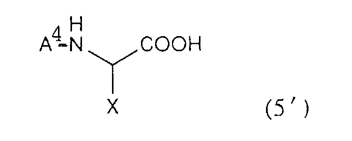 wherein A⁴ is Ar-O-CH₂-CO and A², Ar, R¹, R², R³ and X are as defined in Claim 1.

3. A process according to claim 1 wherein amino acid derivative (5) is of formula 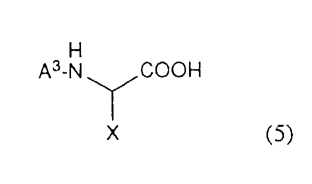 (wherein X is methylthiomethyl and A³ is *tert*-butoxycarbonyl) and is obtained by methylation of cysteine and subsequent one-pot amino-protection using di-tert-butyl dicarbonate.

4. A process according to any preceding claim wherein additive of said carbodiimide-additive method is a hydroxyl compound selected from 1-hydroxybenzotriazole, N-hydroxysuccinimide, N-hydroxy-5-norbornene-2,3-dicarboximide, and 3-hydroxy-4-oxo-3,4-dihydro-1,2,3-benzotriazine.

5. A process for producing amino acid derivative of formula (6) 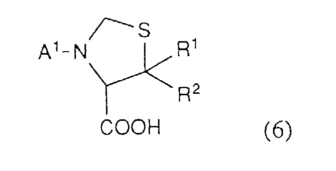 wherein A¹ represents *tert*-butoxycarbonyl which comprises cyclization of compound of formula (13) 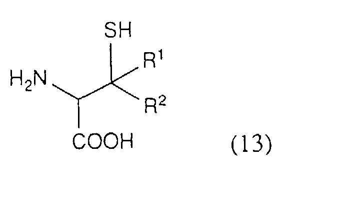 or salt thereof using formaldehyde and subsequent one-pot amino-protection using di-*tert*-butyl dicarbonate, wherein R¹ and R² are as defined in claim 1.

6. A process for producing amino acid derivative of formula (5) 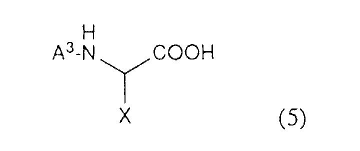 wherein X is methylthiomethyl and A³ represents *tert*-butoxycarbonyl which comprises methylation of cysteine and subsequent one-pot amino-protection using di-*tert*-butyl dicarbonate.

7. A process for producing amino amide of formula (9) 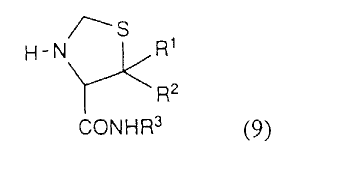 wherein R³ is as defined in claim 1 which comprises producing a protected amino acid of formula (6) by a process according to claim 5 condensation of said protected amino acid with amine of formula (7)
H₂N-R³ (7)
to give a protected amino amide of formula (8) 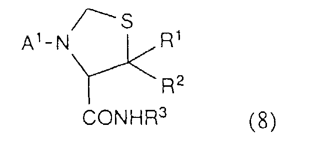 and deprotection of said protected amino amide to give said amino amide of formula (9).

## Patentansprüche

1. Verfahren zur Herstellung eines Peptid-Derivates (1) oder eines Salzes davon, 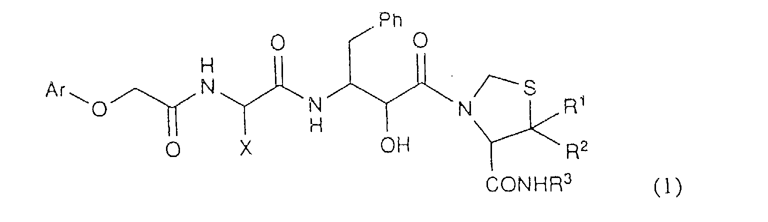 umfassend die folgenden Schritte: Kondensieren eines Aminoamids der Formel (9) 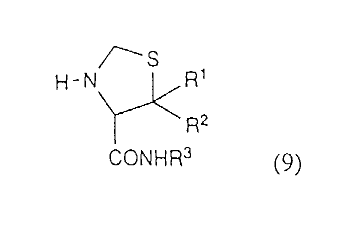 mit einer geschützten Aminosäure der Formel (10) 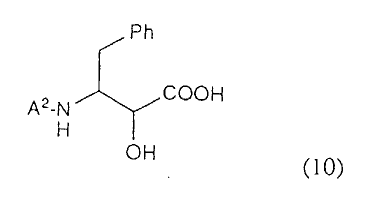 unter Anwendung des Carbodiimid-Additionsverfahrens, um ein geschütztes Peptid der Formel (11) zu erhalten, 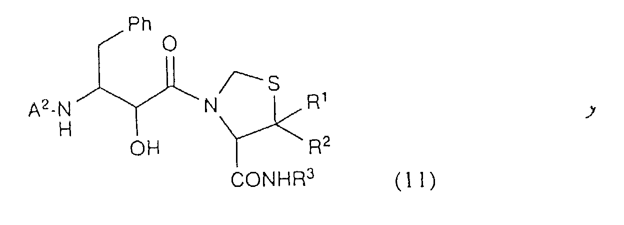 Aufheben des Schutzes des genannten geschützten Peptides, um ein Peptid der Formel (4) zu erhalten, 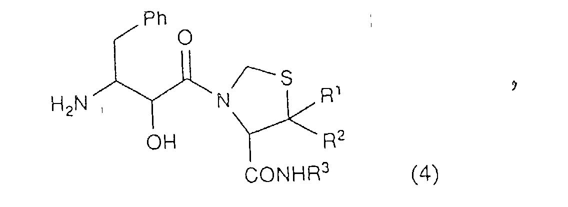 Kondensieren des genannten Peptides (4) mit einer geschützten Aminosäure der Formel (5), 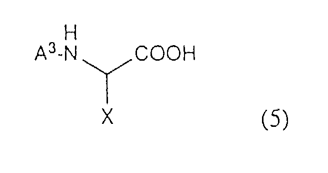 um ein geschütztes Peptid der Formel (12) zu erhalten, 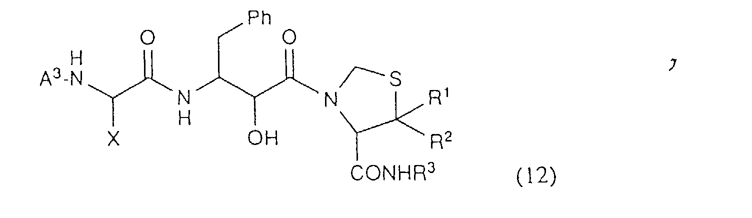 Aufheben des Schutzes des geschützten Peptides (12), um ein Peptid der Formel (2) zu erhalten 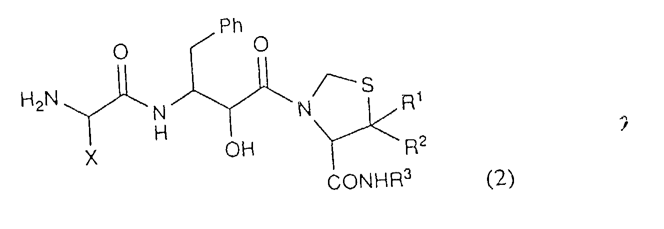 und Kondensieren des Peptides (2) mit Carbonsäure der Formel (3), 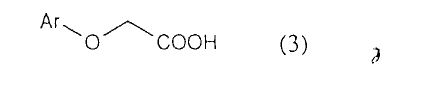 wobei A² und A³ Aminoschutzgruppen sind, R¹ und R² ausgewählt werden aus C₁-C₆-Alkylgruppen und einem Wasserstoffatom, R³ eine C₁-C₆-Alkylgruppe ist, X ein Methylthiomethyl, Methansulfonylmethyl, Carbamoylmethyl oder eine C₁-C₆-Alkylgruppe ist und Ar eine Aryl- oder Heteroarylgruppe ist.

2. Verfahren zur Herstellung eines Peptid-Derivates (1) oder eines Salzes davon, 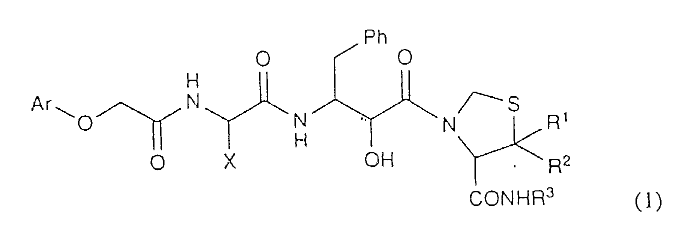 umfassend die folgenden Schritte: Kondensieren eines Aminoamids der Formel (9) 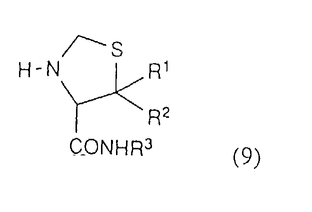 mit einer geschützten Aminosäure der Formel (10) 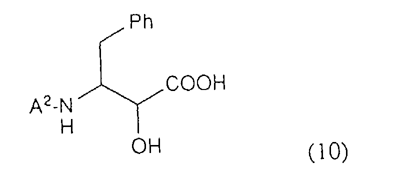 unter Anwendung des Carbodiimid-Additionsverfahrens, um ein geschütztes Peptid der Formel (11) zu erhalten, 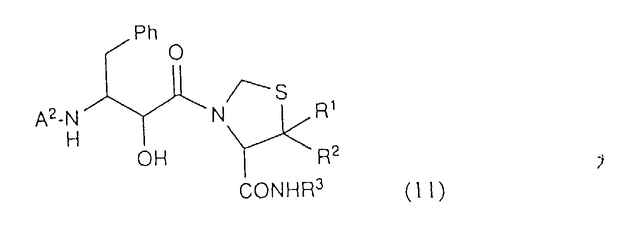 Aufheben des Schutzes des genannten geschützten Peptides, um ein Peptid der Formel (4) zu erhalten 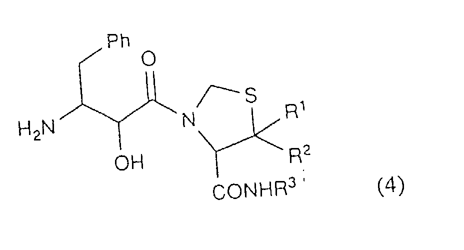 und Kondensieren des Peptids (4) mit einer geschützten Aminosäure der Formel (5') 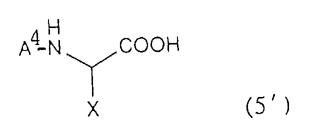 wobei A⁴ Ar-O-CH₂-CO ist und A², Ar, R¹, R², R³ und X der Definition in Anspruch 1 entsprechen.

3. Verfahren nach Anspruch 1, bei dem das Aminosäure-Derivat (5) die folgende Formel hat 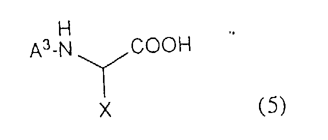 (wobei X Methylthiomethyl und A³ *Tert*-Butoxycarbonyl ist) und durch Methylierung von Cystein und anschließendem Einzeltopf-Aminoschutz unter Verwendung von Di-*Tert*-Butyl-Dicarbonat erzeugt wird.

4. Verfahren nach einem der vorherigen Ansprüche, bei dem der Zusatzstoff des genannten Carbodiimid-Additionsverfahrens eine Hydroxylverbindung ist, ausgewählt aus 1-Hydroxybenzotriazol, N-Hydroxysuccinimid, N-Hydroxy-5-Norbornen-2,3-Dicarboximid und 3-Hydroxy-4-Oxo-3,4-Dihydro-1,2,3-Benzotriazin.

5. Verfahren zur Herstellung eines Aminosäure-Derivates der Formel (6), 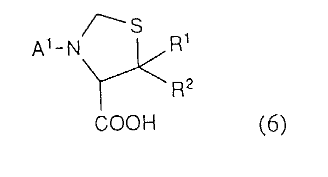 wobei A¹ *Tert*-Butoxycarbonyl repräsentiert, umfassend das Cyclisieren einer Verbindung der Formel (13) 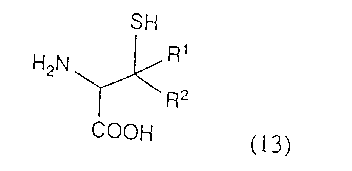 oder eines Salzes davon unter Verwendung von Formaldehyd und den anschließenden Einzeltopf-Aminoschutz unter Verwendung von Di-*Tert*-Butyl-Dicarbonat, wobei R¹ und R² der Definition in Anspruch 1 entsprechen.

6. Verfahren zur Herstellung eines Aminosäure-Derivates der Formel (5), 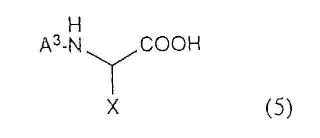 wobei X Methylthiomethyl ist und A³ *Tert*-Butoxycarbonyl repräsentiert, umfassend das Methylieren von Cystein und den anschließenden Einzeltopf-Aminoschutz unter Verwendung von Di-*Tert*-Butyl-Dicarbonat.

7. Verfahren zur Herstellung eines Aminoamides der Formel (9), 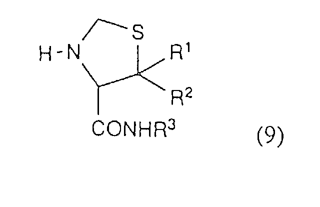 wobei R³ der Definition in Anspruch 1 entspricht, umfassend die folgenden Schritte: Herstellen einer geschützten Aminosäure der Formel (6) mit einem Verfahren nach Anspruch 5,
Kondensieren der genannten geschützten Aminosäure mit einem Amin der Formel (7),
H₂N-R³ (7)
um ein geschütztes Aminoamid der Formel (8) zu erhalten, 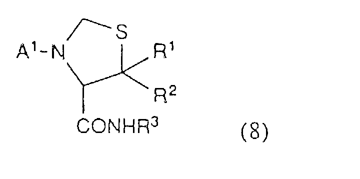 und Aufheben des Schutzes des genannten geschützten Aminoamids, um das genannte Aminoamid der Formel (9) zu erhalten.

## Revendications

1. Un procédé pour produire un dérivé peptide (1) ou sel de celui-ci 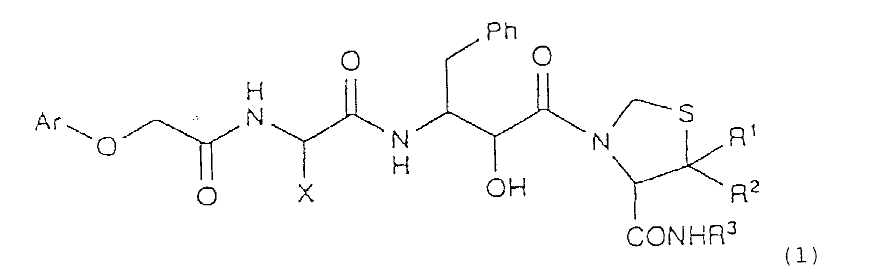 qui comprend la condensation d'un amino-amide de la formule (9) 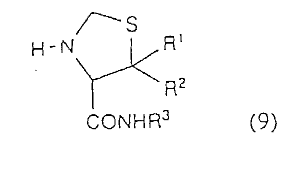 avec un amino-acide protégé de la formule (10) 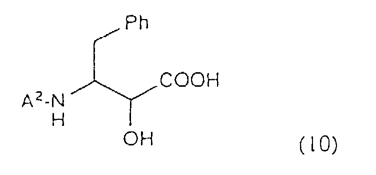 par une méthode à carbodiimide d'addition pour donner un peptide protégé de la formule (11) 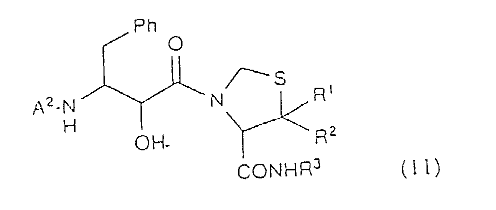 la déprotection dudit peptide protégé pour donner un peptide de la formule (4) 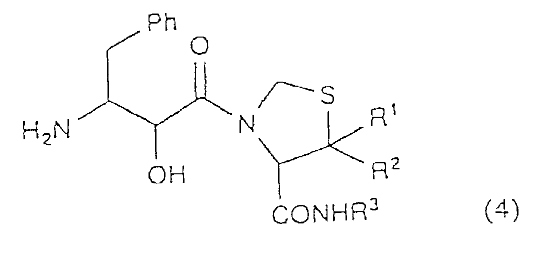 la condensation dudit peptide (4) avec un amino-acide protégé de la formule (5) 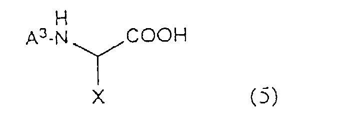 pour donner un peptide protégé de la formule (12) 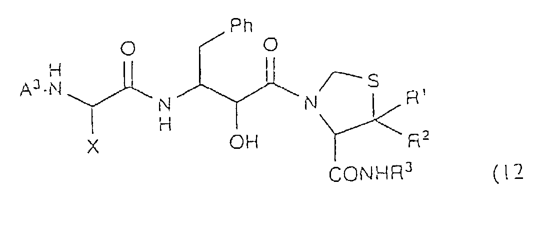 la déprotection du peptide protégé (12) pour donner un peptide de la formule (2) 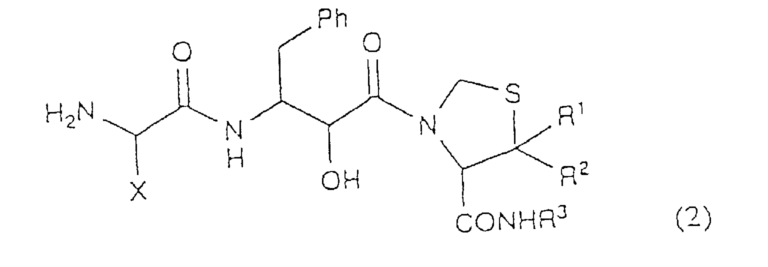 et la condensation du peptide (2) avec de l'acide carboxylique de la formule (3) 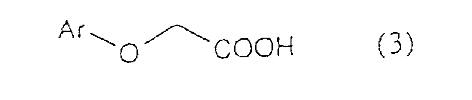 dans laquelle A² et A³ sont des groupes amino-protecteurs, R¹ et R² sont sélectionnés à partir de groupes alkyles C₁-C₆ et d'un atome d'hydrogène, R³ est un groupe alkyle C₁-C₆, X est un groupe méthylthiométhyle, méthanesulfonylméthyle, carbamoylméthyle ou alkyle C₁-C₆ et Ar est un groupe aryle ou hétéro-aryle.

2. Un procédé pour produire un dérivé peptide (1) ou sel de celui-ci 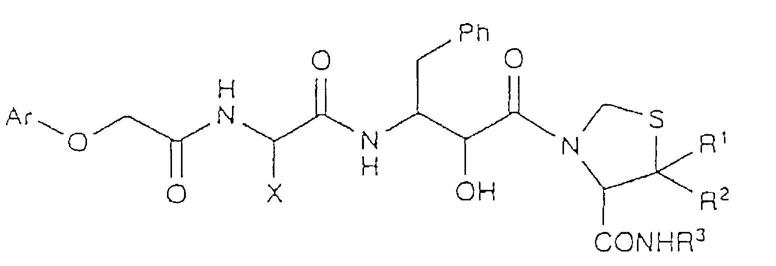 qui comprend la condensation d'un amino-amide de la formule (9) 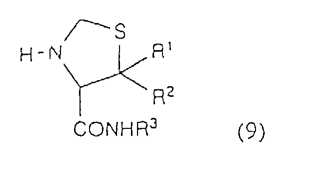 avec un amino-acide protégé de la formule (10) 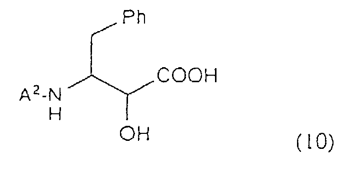 par une méthode à carbodiimide d'addition pour donner un peptide protégé de la formule (11) 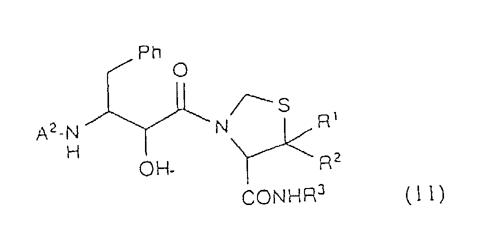 la déprotection dudit peptide protégé pour donner un peptide de la formule (4) 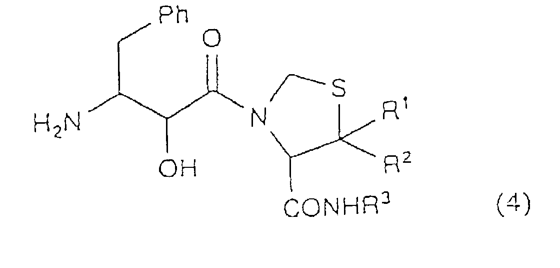 et la condensation du peptide (4) avec un amino-acide protégé de la formule (5') 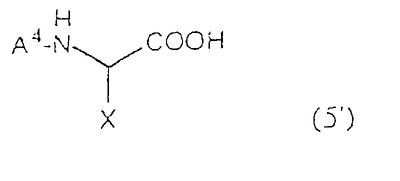 dans laquelle A⁴ est Ar-O-CH₂-CO et A², Ar, R¹, R², R³ et X sont tels que définis dans la revendication 1.

3. Un procédé selon la revendication 1 dans lequel le dérivé amino-acide (5) est de la formule 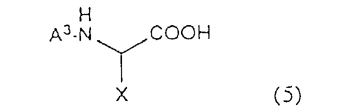 (dans laquelle X est du méthylthiométhyle et A³ est du tert-butoxycarbonyle) et est obtenu par méthylation de cystéine et amino-protection subséquente en un seul appareil en utilisant du dicarbonate de di-*tert*-butyle.

4. Un procédé selon l'une quelconque des revendications précédentes, dans lequel l'additif de ladite méthode à carbodiimide d'addition est un composé hydroxyle sélectionné à partir d'hydroxy-benzotriazole-1, de N-hydroxy-succinimide, de N-hydroxy-norbornène-5-dicarboximide -2,3, et d'hydroxy-3-oxo-4-dihydro-3,4-benzotriazine-1,2,3.

5. Un procédé pour produire un dérivé amino-acide de la formule (6) 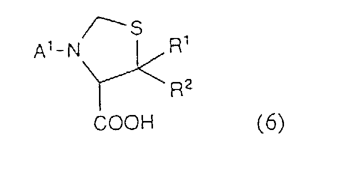 dans laquelle A¹ représente du *tert*-butoxycarbonyle qui comprend la cyclisation du composé de la formule (13) 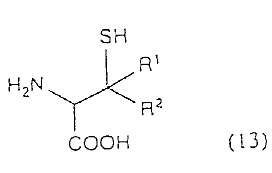 ou du sel de celui-ci en utilisant du formaldéhyde et une amino-protection subséquente en un seul appareil en utilisant du dicarbonate de di-*tert*-butyle, dans laquelle R¹ et R² sont tels que définis dans la revendication 1.

6. Un procédé pour produire un dérivé amino-acide de la formule (5) 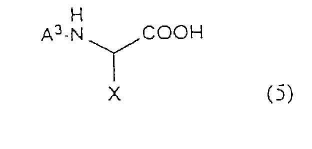 dans laquelle X est du méthylthiométhyle et A³ représente du *tert*-butoxycarbonyle qui comprend méthylation de cystéine et amino-protection subséquente en un seul appareil en utilisant du dicarbonate de di-*tert*-butyle.

7. Un procédé pour produire un amino-amide de la formule (9) 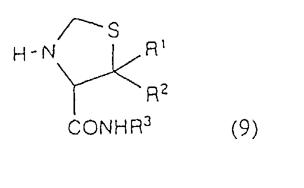 dans laquelle R³ est tel que défini dans la revendication 1 qui comprend la production d'un amino-acide protégé de la formule (6) par un procédé conforme à la revendication 5
la condensation dudit amino-acide protégé avec une amine de la formule (7)
H₂N-R³ (7)
pour donner un amino-amide protégé de la formule (8) 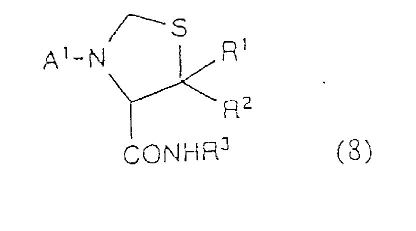 et la déprotection dudit amino-amide protégé pour donner ledit amino-amide de la formule (9).
